# FASCICULE DE BREVET EUROPEEN

(11) **EP 1 582 196 B1**
(45) Date de publication et mention de la délivrance du brevet: **24.08.2011**
(21) Numéro de dépôt: 05013409.7
(22) Date de dépôt: 14.01.2002
(51) Int. Cl.: A61K 8/06, A61K 8/44

(54) **Nanoémulsion translucide, son procédé de fabrication et ses utilisations dans les domaines cosmétique, dermatologique et/ou ophtalmologique**
Durchscheinende Nanoemulsion, Verfahren zur deren Herstellung, und deren Verwendungen in Kosmetika, Dermatologie und/oder Ophtalmologie.
Translucent nanoemulsion, production method, and uses thereof in the cosmetic, dermatological and/or ophthalmological fields.

(30) Priorité: 18.01.2001 FR 0100696
(43) Date de publication de la demande: 05.10.2005
(62) Demande divisionnaire de: 02711954.4
(73) Titulaire: L'Oréal, 75008 Paris (FR)
(72) Inventeur: Quemin, Eric, 94340 Joinville le Pont (FR)
(74) Mandataire: Duvert, Sandra

(56) Documents cités:
- EP-A- 0 790 297
- EP-A- 0 968 704
- WO-A-96/00557
- FR-A- 2 005 465
- GB-A- 801 119
- US-A- 6 013 270

## Description

La présente invention concerne une nanoémulsion translucide, stable, à base d'un système ternaire de tensioactifs, ne nécessitant ni l'emploi d'alcools inférieurs et/ou de polyols, qui limitent les applications sur peaux sensibles, ni d'agents gélifiants pour la stabilisation.

L'invention se rapporte également à un procédé de préparation de ladite nanoémulsion et à ses utilisations dans les domaines cosmétique, dermatologique et/ou ophtalmologique. Cette nanoémulsion est stable au stockage et peut contenir des quantités importantes d'huiles tout en conservant une bonne transparence et en ayant de bonnes propriétés cosmétiques.

Les nanoémulsions sont des émulsions huile dans eau dont les globules d'huile ont une granulométrie très fine, c'est-à-dire une taille moyenne en nombre, inférieure à 100 nanomètres (nm). Elles sont généralement fabriquées par fragmentation mécanique d'une phase huileuse dans une phase aqueuse en présence de tensioactifs. Dans le cas des nanoémulsions, la très petite taille des globules huileuses est obtenue notamment grâce à au moins un passage dans un homogénéiseur haute pression. La petite taille des globules leur confère des propriétés intéressantes sur le plan cosmétique qui les distinguent des émulsions classiques : elles sont translucides, voire transparentes et présentent une texture originale. Elles peuvent également véhiculer des actifs de façon plus efficace.

On connaît dans l'état de la technique des nanoémulsions comprenant une phase lipidique amphiphile constituée de phospholipides, d'eau et d'huile. Ces émulsions présentent l'inconvénient d'être instables au stockage aux températures traditionnelles de conservation, à savoir entre 0 et 45°C. Elles conduisent à des compositions jaunes et produisent des odeurs de rance qui se développent après quelques jours de conservation.

On connaît également, par exemple du document EP0705593, des nanoémulsions stabilisées par un enrobage cristal liquide lamellaire obtenu par l'association d'un tensioactif hydrophile et d'un tensioactif lipophile. Toutefois, ces associations sont délicates à déterminer. De plus, les nanoémulsions obtenues présentent un toucher cireux et filmogène, peu agréable pour l'utilisateur.

La demande de brevet international WO 98/47464 décrit une lotion stable, ayant essentiellement la consistance de l'eau et qui convient pour une lotion pulvérisable. Cette lotion, une émulsion homogénéisée à haute pression ne nécessite pas d'agents épaississants ou stabilisants. L'émulsion est faite en deux temps. Dans un premier temps, on forme un prémix concentré dont les émulsionnants sont des éthoxylates d'alcool stéarylique. Le prémix concentré est alors homogénéisé à 1000 bars. L'émulsion concentrée obtenue est ensuite diluée à 50% dans de l'eau au moyen d'équipements classiques de mélange, afin d'obtenir une lotion. Cette technique présente l'inconvénient de comporter une étape de dilution.

Si l'on cherche à augmenter la proportion de la phase grasse dans une émulsion pour se rapprocher de la consistance d'un gel ou d'une crème, il n'existe pas, à l'heure actuelle, d'exemples de formulation à la fois translucides et consistantes qui ne font pas recours à des alcools inférieurs (alcools en C₁-C₈) ou à des polyols (glycol et polyalkylène glycol) pour affiner la taille des globules d'huile dans l'émulsion, ce qui ne permet pas de les utiliser pour des peaux sensibles et à des gélifiants pour stabiliser les systèmes par un apport de consistance.

La demande de brevet européen EP-728.460 décrit des nanoémulsions contenant deux types de tensioactifs :
- un tensioactif non ionique sélectionné parmi les esters gras de polyéthylène glycol ou de sorbitol ;
- un tensioactif ionique, dont le dicétylphosphate de potassium.

Pour obtenir une nanoémulsion transparente, il faut ajouter 5 à 20% en poids d'éthanol (qui est un composé potentiellement pro-irritant), afin d'affiner la granulométrie des globules d'huile.

Par ailleurs, les demandes de brevet EP-1.016.453 et EP-1.010.415 décrivent également une nanoémulsion contenant des alcools inférieurs ou des polyols (éthanol, dipropylène glycol, polyéthylène glycol) pour affiner la granulométrie.
La présente invention a donc pour objet de fournir une nanoémulsion comportant une phase huileuse dispersée dans une phase aqueuse qui soit translucide.

La présente invention a encore pour objet une nanoémulsion translucide comportant une phase huileuse dispersée dans une phase aqueuse qui ne comprend pas d'alcools inférieurs et/ou de polyols, permettant ainsi son application aux peaux sensibles.

La présente invention a aussi pour objet une nanoémulsion telle que définie ci-dessus ayant la consistance d'un gel ou d'une crème et qui de préférence ne comporte pas d'agents de gélification.

La présente invention a également pour objet une nanoémulsion telle que définie précédemment qui est stable vis-à-vis du mûrissement, même en l'absence d'alcools inférieurs et/ou de polyols et/ou d'agents de gélification.

Selon l'invention, on réalise une nanoémulsion comportant une phase huileuse dispersée dans une phase aqueuse ayant des globules d'huile dont la taille moyenne en nombre est inférieure à 100 nm, caractérisée en ce qu'elle contient un système ternaire de tensioactifs comprenant :
a) un mélange d'au moins deux tensioactifs non ioniques comprenant au moins un ester gras éthoxylé comportant 8 à 100 unités d'oxyde d'éthylène et au moins un ester d'acide gras de sorbitane ; et
b) au moins un tensioactif ionique choisi parmi les sels alcalins de palmitoyl sarcosinate.

Les nanoémulsions selon l'invention ont généralement un aspect translucide à transparent et éventuellement une légère coloration, par exemple une légère coloration rosée ou bleutée. Elles ont généralement une turbidité allant de 60 à 600 NTU, mesurée au turbidimètre portatif HACH - Modèle 2100 P.

Les globules d'huile des nanoémulsions selon l'invention ont une taille moyenne en nombre, inférieure à 100 nm et de préférence, de 50 à 90 nm. Cette taille de globules peut être mesurée par exemple avec un appareil BROOKHAVEN BI 90 et déterminée selon la méthode connue de « Diffusion quasi élastique de la lumière ». La diminution de la taille des globules permet de favoriser la concentration des actifs dans les couches superficielles de la peau (effet véhicule).

Le système ternaire de tensioactifs utilisable dans la nanoémulsion de l'invention comprend, comme indiqué ci-dessus, un premier constituant essentiel (a) qui est un mélange d'au moins deux tensioactifs non ioniques comprenant au moins un ester gras éthoxylé comportant 8 à 100 unités d'oxyde éthylène et au moins un ester d'acide gras de sorbitane.

La chaîne grasse des esters du mélange (a) comportent généralement de 16 à 22 atomes de carbone. La chaîne grasse des esters peut être notamment choisie parmi les motifs stéaryle, béhényle, arachidyle, palmityle, cetyle et leurs mélanges tel que cétéaryle.

Le nombre d'unités d'oxyde d'éthylène va de 8 à 100, de préférence de 10 à 80, et mieux de 20 à 60. Selon un mode particulier de réalisation de l'invention, ce nombre est de 40.

A titre d'exemple d'ester gras éthoxylé ayant 40 unités d'oxyde d'éthylène, on peut citer l'ester d'acide stéarique comprenant 40 unités d'oxyde d'éthylène, tel que le produit commercialisé sous la dénomination Myrj 52 (stéarate de polyéthylèneglycol 40 OE ; nom CTFA : PEG-40 stéarate) par la société UNIQEMA.

A titre d'exemple d'ester d'acide gras de sorbitane, on peut citer le tristéarate de sorbitane.

De préférence, le mélange (a) de tensioactifs non ioniques comprend un ester gras éthoxylé, en particulier le PEG-40 stéarate, et un ester de sorbitane, en particulier le tristéarate de sorbitane.

En général, le rapport pondéral de l'ester gras éthoxylé 8 à 100 OE à l'ester de sorbitane du mélange (a) varie de 0,02 à 100, de préférence de 0,04 à 80.

En général, l'ester gras éthoxylé 8 à 100 OE représente 0,01 à 10 % en poids, de préférence de 0,1 à 5 % en poids, et mieux de 0,5 à 3 %, par rapport au poids total de la nanoémulsion.

L'ester d'acide gras de sorbitane représente généralement 0,1 % à 10 % en poids, et de préférence 0,5 à 5 % en poids, par rapport au poids total de la nanoémulsion.

Le deuxième constituant essentiel du système ternaire de tensioactifs (b) comprend au moins un tensioactif ionique choisi parmi les , les sels alcalins de palmitoyl sarcosinate .

En général, le rapport du constituant (b) au constituant (a) du système ternaire de tensioactifs varie de 0,02 à 75, de préférence de 0,02 à 10.

La teneur en tensioactif ionique selon l'invention peut varier de 0,05 à 10 % en poids, et de préférence de 0,2 à 5 % et mieux de 0,5 à 3 % en poids, par rapport au poids total de la nanoémulsion.

La nanoémulsion selon l'invention comporte une phase huileuse. Typiquement, le rapport pondéral du système ternaire de tensioactif à la phase huileuse varie de 6.10⁻³ à 60, de préférence de 0,4 à 19.

Généralement, la phase huileuse représente de 0,5 % à 40 % en poids, et de préférence de 5 à 30 % en poids, par rapport au poids total de la nanoémulsion.

La phase huileuse des nanoémulsions selon l'invention contient au moins une huile qui peut être choisie parmi les huiles d'origine animale ou végétale, les huiles minérales, les huiles de synthèse, les huiles de silicone, les hydrocarbures notamment aliphatiques, et leurs mélanges. Ces huiles peuvent être polaires ou non polaires, volatiles ou non volatiles.

Parmi les huiles polaires, on peut citer les huiles hydrocarbonées comportant des fonctions ester, éther, acide, alcool ou leurs mélanges, telles que par exemple :
- les huiles végétales hydrocarbonées à forte teneur en triglycéride constituées d'ester d'acide gras et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépin de raisin ou de cassis, d'onagre, de millet, d'orge, de quinoa, d'olive, de seigle, de carthame, de bancoulier, de passiflore, de rosier muscat ou encore les triglycérides des acides capryliques / capriques comme ceux vendus par la société STEARINERIES DUBOIS ou ceux vendus sous une dénomination MIGLYOL 810, 812 et 818 par la société DYNAMIT NOBEL ;
   ● les huiles de synthèse de formule R¹COOR², dans laquelle R¹ représente le reste d'un acide gras supérieur, linéaire ou ramifié, comportant de 7 à 19 atomes de carbone, et R² représente une chaîne hydrocarbonée ramifiée contenant de 3 à 20 atomes de carbone, comme par exemple l'huile de Purcellin (octanoate de cétostéaryle), l'isononoate d'isononyle, les benzoates d'alkyle (en C₁₂ à C₁₅) ;
   ● les esters et les éthers de synthèse comme le myristate d'isopropyle, le palmitate d'éthyl-2-hexyle, les octanoates, les décanoates ou les ricinoléates d'alcools ou de polyalcools ;
   ● les esters hydroxylés comme le lactate d'isostéaryle, le malate de diisostéaryle, et les esters de pentaérythritol.

Parmi les huiles apolaires, on peut citer :
● les huiles de silicones volatiles ou non, linéaires ou cycliques, liquides à température ambiante, telles que les polydiméthylsiloxanes (PDMS) comportant des groupements alkyle, alcoxy ou phényle, pendants et/ou en bout de chaîne siliconée et ayant de 2 à 24 atomes de carbone ; les silicones phénylées, comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates,
● les hydrocarbures ou les hydrocarbures fluorés/ou fluorocarbures, linéaires ou ramifiés d'origine synthétique ou minérale, comme les huiles volatiles, telles que les huiles de paraffine (par exemple les isoparaffines), et les hydrocarbures aliphatiques (par exemple l'isododécane), ou non volatiles et leurs dérivés, la vaseline, les polydécènes, le polyisobutène hydrogéné tel que l'huile de Parléam, le squalane, et leurs mélanges.
L'huile apolaire préférée est l'huile de Parléam.

La phase huileuse peut également comporter des corps gras autres que les huiles indiquées ci-dessus, tels que un ou plusieurs alcools gras comme les alcools stéarylique, cétylique, béhénique, les acides gras comme les acides stéarylique, palmitique et béhénique, les cires tels que des mono, di, tri, palmitostéarates de glycéryle, les gommes et leurs mélanges.

Lorsqu'il est présent, cet autre corps gras, de préférence l'alcool cétylique, peut représenter par exemple jusqu'à 10% en poids, de préférence de 2 à 5% en poids, du poids total de la nanoémulsion.

Bien que les nanoémulsions conformes à la présente invention puissent contenir des additifs pour améliorer la transparence de la formulation, tels que des alcools inférieurs en C₁-C₈ comme l'éthanol, les glycols tels que la glycérine, le propylène glycol, le dipropylène glycol, les nanoémulsion, selon l'invention, sont de préférence exemptes de ces additifs qui sont généralement pro-irritants.

Bien que les nanoémulsions, selon l'invention, puissent comporter des agents de gélification, tels que les dérivés de cellulose, les dérivés d'algues, les gommes naturelles, les polymères synthétiques tels que les mélanges d'acides polycarboxyvinyliques, les nanoémulsions selon l'invention sont de préférences exemptes de ce type d'agents de gélification.

Les nanoémulsions, selon l'invention, ont généralement la consistance d'un gel ou d'une crème. La viscosité des nanoémulsions, conformes à l'invention, varie généralement de 1 à 30 poises (= 0,1 à 3 Pa.s), de préférence de 5 à 20 poises (= 0,5 à 2 Pa.s), ces viscosités étant mesurées à 25°C avec un viscosimètre Rhéomat 180 (mobile 3).

Les nanoémulsions, selon l'invention, peuvent également comporter les additifs classiquement utilisés en cosmétologie, tels que des conservateurs, comme les alkylparabens, des parfums, et des pigments, en particulier en vue de l'application maquillage, fond de teint, eye-liner, etc.

Les nanoémulsions de l'invention conservent une excellente stabilité après les deux mois de vieillissement accéléré à 4°C, température ambiante, et 45°C.

Les nanoémulsions définies ci-dessus peuvent être utilisées dans tous domaines où ce type de composition est utile. Elles peuvent constituer notamment des compositions à usage topique, notamment cosmétiques et dermatologiques Elles peuvent aussi être utilisées comme supports ophtalmiques Elles peuvent en outre constituer dans le domaine pharmaceutique une composition administrable par voie orale, parentérale ou transcutanée.

Un autre objet de l'invention consiste donc en une composition à usage topique, caractérisée en ce qu'elle contient une nanoémulsion telle que définie précédemment.

L'invention a aussi pour objet un support ophtalmique, caractérisé en ce qu'elle contient une nanoémulsion telle que définie précédemment.

Les nanoémulsions de l'invention peuvent contenir des actifs hydrosolubles ou liposolubles ayant une activité cosmétique, dermatologique ou ophtalmologique.

Les actifs liposolubles sont dans les globules huileux de l'émulsion, tandis que les actifs hydrosolubles sont dans la phase aqueuse de l'émulsion. On peut citer, à titre d'exemples d'actifs, les vitamines, telles que la vitamine E, la vitamine C, la vitamine A, la vitamine PP et leurs dérivés et, en particulier, leurs esters, les pro-vitamines telles que le panthénol, les humectants et les filtres solaires.

Comme actifs ophtalmiques on peut citer par exemple les agents antiglaucome tels que le betaxolol ; les antibiotiques tels que l'acyclovir ; les antiallergiques ; les agents anti-inflammatoires tels que l'ibuprofène et ses sels, le diclofénac et ses sels, l'indométhacine ; les agents antiviraux.

L'invention a aussi pour objet un procédé de préparation d'une nanoémulsion telle que définie ci-dessus, procédé consistant à mélanger la phase aqueuse et la phase huileuse sous agitation vive, à une température allant de 60 à 95°C, puis à effectuer une homogénéisation à une pression allant, de préférence, de 6.10⁷ Pa à 18.10⁷ Pa (homogénéisation haute pression).

Le cisaillement va de préférence de 2.10⁶ s⁻¹ à 5.10⁸ s⁻¹ et mieux de 1.10⁸ s⁻¹ à 35.10⁸ s⁻¹ .

La nanoémulsion de l'invention peut être par exemple utilisée pour le soin, le traitement, le maquillage de la peau, du visage et/ou du cuir chevelu.

L'invention a donc aussi pour objet l'utilisation cosmétique de la nanoémulsion telle que définie ci-dessus pour le soin, le traitement et/ou le maquillage de la peau, du visage et/ou du cuir chevelu.

En outre, la nanoémulsion de l'invention peut être aussi utilisée pour le soin et/ou le traitement des cheveux. Elle permet d'obtenir un dépôt d'huile sur les cheveux, ce qui rend ceux-ci plus brillants, plus résistants au coiffage, sans toutefois les alourdir. Elle permet aussi, en prétraitement, d'améliorer les effets de la coloration ou de la permanente. L'invention a donc aussi pour objet l'utilisation cosmétique de la nanoémulsion telle que définie ci-dessus pour le soin et/ou le traitement des cheveux.

La nanoémulsion, selon l'invention, permet notamment une bonne hydratation de la peau, des muqueuses et/ou du cuir chevelu et est particulièrement adaptée au traitement de la peau sèche.

Un autre objet de l'invention est donc un procédé cosmétique de soin et/ou d'hydratation de la peau, des muqueuses et/ou du cuir chevelu, caractérisé en ce qu'on applique sur la peau, les muqueuses et/ou le cuir chevelu, une nanoémulsion telle que définie ci-dessus.

L'invention porte enfin sur l'utilisation de la nanoémulsion selon l'invention pour la fabrication d'une composition dermatologique ou ophtalmologique, notamment pour la fabrication d'une composition dermatologique destinée au traitement de la peau sèche.

Les exemples qui suivent, permettront de mieux comprendre l'invention, sans toutefois présenter un caractère limitatif. Dans les exemples, sauf indication contraire, les pourcentages et parties sont exprimés en poids.

Les nanoémulsions des exemples 1 et 2 ci-dessous ont été obtenues en formant une pré-émulsion grossière au rotor-stator, en ajoutant la phase aqueuse A sur la phase huileuse B, à 80°C. Le prémix était ensuite passé cinq fois dans un homogénéisateur haute pression (Soavi type OBL 20) avec une pression au premier étage de 1100 bars et une pression au second étage de 120 bars, avec un refroidissement à 70°C en sortie.

### Exemple 1

| | | |
|---|---|---|
| **A** | eau | 72.60% |
| | méthylparaben | 0.2% |
| **B** | Tri stéarate de sorbitane | 0.9% |
| | Alcool cétylique | 4% |
| | Mono,di,tri palmito stéarate de glycéryle | 3.3% |
| | Stéarate de polyéthylène glycol (40 OE) | 2% |
| | Huile de Parléam | 15.95% |
| | Cétyl phosphate de potassium | 0.75% |
| | propylparaben | 0.1 % |
| | Parfum | 0.2% |

### Exemple 2

| | | |
|---|---|---|
| **A** | eau | 72.60% |
| | méthylparaben | 0.2% |
| **B** | Tri stéarate de sorbitane | 0.9% |
| | Alcool cétylique | 4% |
| | Mono,di,tri palmito stéarate de glycéryle | 3.3% |
| | Stéarate de polyéthylène glycol (40 OE) | 2% |
| | Huile de Parléam | 15.95% |
| | Palmytoyl sarcosinate de sodium | 0.75% |
| | propylparaben | 0.1 % |
| | Parfum | 0.2% |

A titre comparatif on a préparé des émulsions comme décrit ci-dessus en remplaçant les tensioactifs ioniques selon l'invention par des tensioactifs ioniques classiquement utilisés en cosmétique. Les résultats sont donnés dans le tableau I ci-dessous :

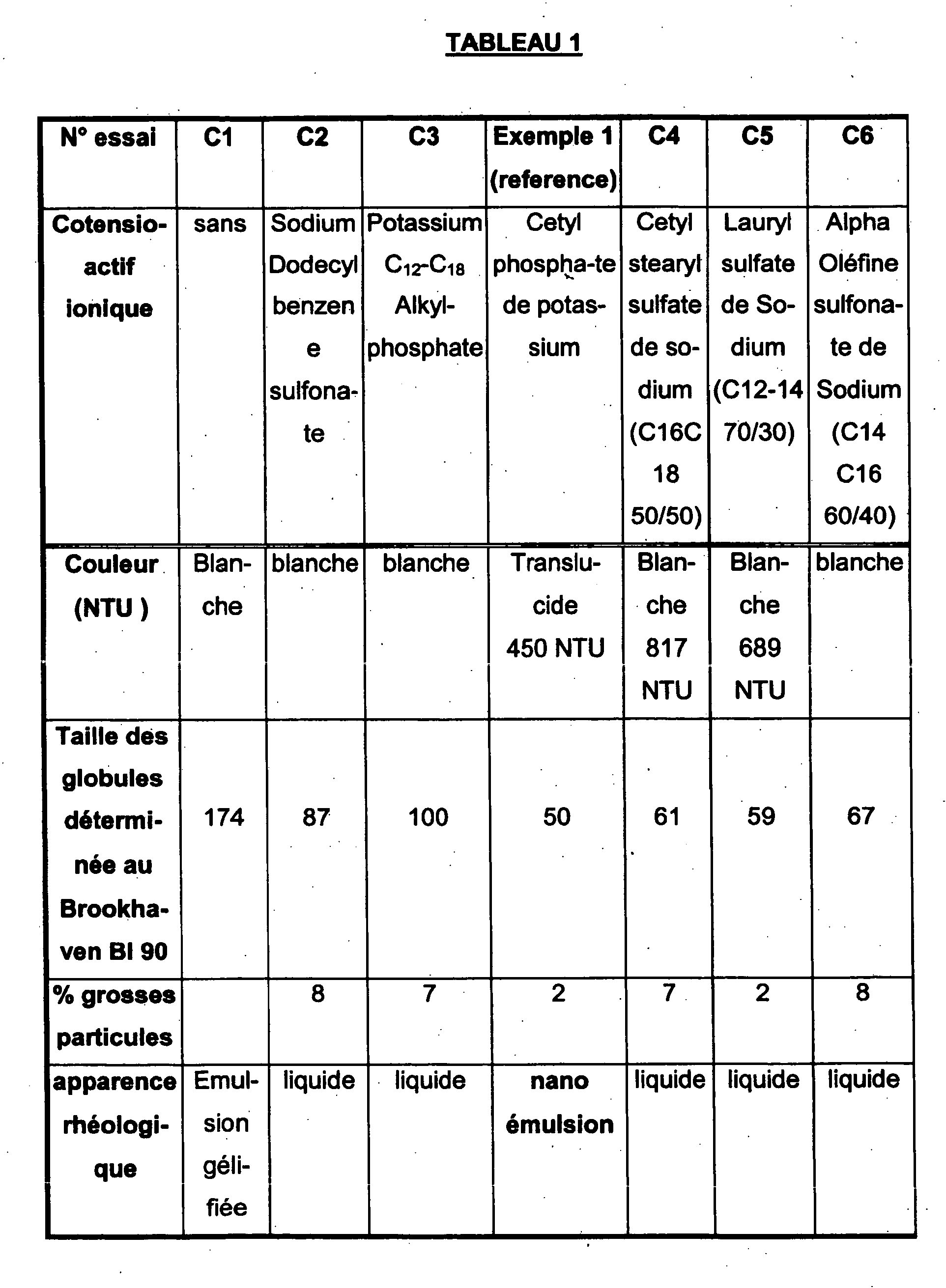

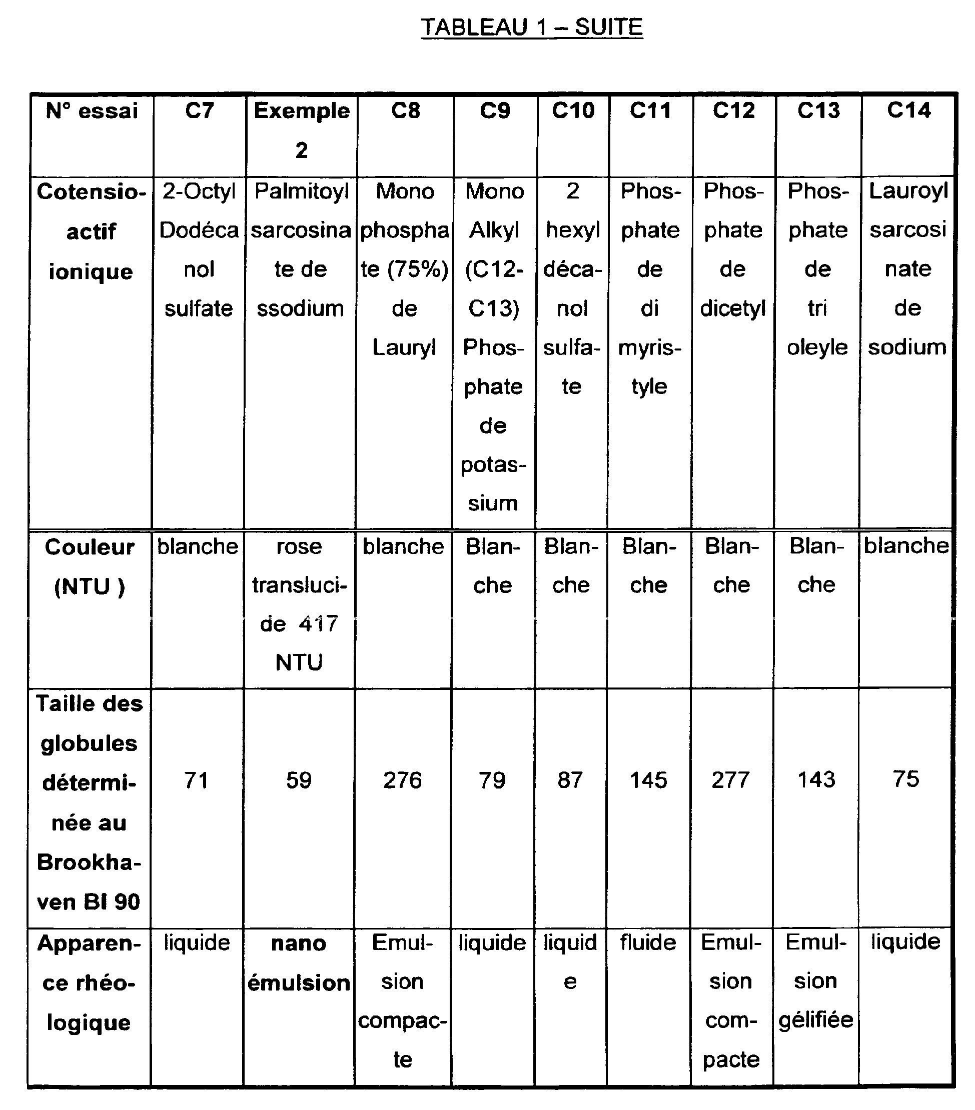

Les résultats du tableau I montrent qu'une nanoémulsion translucide n'est obtenue qu'avec les cotensioactifs ioniques selon l'invention.

La transparence des émulsions et nanoémulsions a été mesurée par un coefficient de transmittance à 600 nm allant de 10 à 90 % ou bien par une turbidité allant de 60 à 600 NTU, turbidité mesurée au turbidimètre portatif HACH-modèle 2100 P (mesure à température ambiante ≃ 25°C).

Les nanoémulsions des exemples 1 et 2 selon l'invention avaient la consistance d'un gel et étaient stables même après les deux mois de vieillissement accéléré à 4°C, température ambiante, et 45°C.

Ces nanoémulsions ont été testées sur un panel de 10 femmes utilisatrices de crème de jour, d'âge moyen de 36 ans. Toutes ont trouvé que les nanoémulsions s'étalaient facilement et pénétraient bien dans la peau, avec une sensation de confort et de fraîcheur se développant immédiatement.

Après application, la peau est douce, souple, hydratée. Aucune des femmes n'a signalé un inconfort quelconque.

## Revendications

1. Nanoémulsion comportant une phase huileuse, dispersée dans une phase aqueuse ayant des globules d'huile dont la taille moyenne en nombre est inférieure à 100 nanomètres, **caractérisée en ce qu'**elle contient un système ternaire de tensioactifs comprenant :
(a) un mélange d'au moins deux tensioactifs non ioniques comprenant au moins un ester gras éthoxylé comportant 8 à 100 unités d'oxyde d'éthylène et au moins un ester d'acide gras de sorbitane ; et
(b) au moins un tensioactif ionique choisi parmi les sels alcalins de palmitoyl sarcosinate.

2. Nanoémulsion selon la revendication 1, **caractérisée en ce que** l'ester gras éthoxylé comprend de 10 à 80 unités d'oxyde d'éthylène.

3. Nanoémulsion selon la revendication 1, **caractérisée en ce que** l'ester gras éthoxylé comprend 40 unités d'oxyde d'éthylène.

4. Nanoémulsion selon l'une quelconque des revendications précédentes, **caractérisée en ce que** l'ester gras éthoxylé est le stéarate de polyéthylèneglycol 40 OE et l'ester d'acide gras de sorbitane est le tristéarate de sorbitane.

5. Nanoémulsion selon l'une quelconque des revendications précédentes, **caractérisée en ce que** le tensioactif ionique est le palmitoyl sarcosinate de sodium.

6. Nanoémulsion selon l'une quelconque des revendications précédentes, **caractérisée en ce que** le rapport pondéral du tensioactif ionique (b) au mélange de tensioactifs non ioniques (a) est tel que :
0,02 ≤ b/a ≤ 75, de préférence 0,02 ≤ b/a ≤ 10.

7. Nanoémulsion selon l'une quelconque des revendications précédentes, **caractérisée en ce que** le rapport pondéral de l'ester gras éthoxylé à l'ester d'acide gras du sorbitane varie de 0,02 à 100, de préférence de 0,04 à 80.

8. Nanoémulsion selon l'une quelconque des revendications précédentes, **caractérisée en ce que** l'ester d'acide gras de sorbitane représente 0,1% à 10% en poids, de préférence 0,5 à 5% en poids, du poids total de la nanoémulsion.

9. Nanoémulsion selon l'une quelconque des revendications précédentes, **caractérisée en ce que** l'ester gras éthoxylé représente de 0,01% à 10% en poids, de préférence 0,1 à 5% en poids, du poids total de la nanoémulsion.

10. Nanoémulsion selon l'une quelconque des revendications précédentes, **caractérisée en ce que** la teneur en tensioactif ionique représente de 0,05% à 10%, et de préférence 0,2% à 5% en poids, du poids total de la nanoémulsion.

11. Nanoémulsion selon l'une quelconque des revendications précédentes, **caractérisée en ce que** la phase huileuse représente de 0,5 à 40% en poids, de préférence de 5 à 30% en poids, par rapport au poids total de la nanoémulsion.

12. Nanoémulsion selon l'une quelconque des revendications précédentes, **caractérisée en ce que** la phase huileuse comprend une huile choisie parmi les huiles d'origine animale ou végétale, les huiles minérales, les huiles de synthèse, les huiles de silicone, les hydrocarbures aliphatiques, et leurs mélanges.

13. Nanoémulsion selon l'une quelconque des revendications précédentes, **caractérisée en ce que** l'huile est un polyisobutène hydrogéné, en particulier l'huile de Parléam.

14. Nanoémulsion selon la revendication 13, **caractérisée en ce qu'**elle comprend, en outre, au moins un autre corps gras choisi parmi les alcools gras, les acides gras, les cires, les gommes et leurs mélanges.

15. Nanoémulsion selon la revendication 14, **caractérisée en ce que** l'autre corps gras représente de 0 à 10 % en poids, de préférence 2 à 5% en poids, du poids total de la nanoémulsion.

16. Nanoémulsion selon la revendication 13 ou 14, **caractérisée en ce que** les alcools gras sont choisis parmi les alcools stéarylique, cétylique, béhénique, et les acides gras sont choisis parmi les acides stéarique, palmitique et béhénique.

17. Nanoémulsion selon la revendication 16, **caractérisée en ce que** l'alcool gras est l'alcool cétylique.

18. Nanoémulsion selon la revendication 14 ou 15, **caractérisée en ce que** l'autre corps gras est le mono, di, tri palmito stéarate de glycéryle.

19. Nanoémulsion selon l'une quelconque des revendications précédentes, **caractérisée en ce qu'**elle contient un actif cosmétique, dermatologique ou ophtalmologique.

20. Composition à usage topique, **caractérisée en ce qu'**elle contient une nanoémulsion selon l'une quelconque des revendications 1 à 19.

21. Utilisation cosmétique de la nanoémulsion selon l'une quelconque des revendications 1 à 18, pour le soin, le traitement et/ou le maquillage de la peau, du visage et/ou du cuir chevelu.

22. Utilisation cosmétique de la nanoémulsion selon l'une quelconque des revendications 1 à 18, pour le soin et/ou le traitement des cheveux.

23. Utilisation de la nanoémulsion selon l'une quelconque des revendications 1 à 18, pour la fabrication d'une composition dermatologique ou ophtalmologique.

24. Procédé cosmétique de soin et/ou d'hydratation de la peau, des muqueuses et/ou du cuir chevelu, **caractérisé en ce qu'**on applique sur la peau, les muqueuses et/ou le cuir chevelu une nanoémulsion selon l'une quelconque des revendications 1 à 18.

25. Procédé de préparation d'une nanoémulsion selon l'une quelconque des revendications 1 à 19 consistant à mélanger la phase aqueuse et la phase huileuse, sous agitation vive, à une température allant de 60 à 95°C, puis à effectuer une homogénéisation à une pression allant de 6.10⁷ Pa à 18.10⁷ Pa.

26. Procédé selon la revendication 25, **caractérisé en ce que** le cisaillement va de 2.10⁶ s⁻¹ à 5.10⁶ s⁻¹.

## Claims

1. Nanoemulsion comprising an oily phase dispersed in an aqueous phase, having oil globules with a numberaverage size less than 100 nm, **characterized in that** it contains a ternary surfactant system comprising:
a) a mixture of at least two nonionic surfactants comprising at least one ethoxylated fatty ester comprising 8 to 100 ethylene oxide units and at least one fatty acid ester of sorbitan; and
b) at least one ionic surfactant chosen from alkali metal salts of palmitoyl sarcosinate.

2. Nanoemulsion according to Clam 1, **characterized in that** the ethoxylated fatty ester comprises from 10 to 80 ethylene oxide units.

3. Nanoemulsion according to Claim 1, **characterized in that** the ethoxylated fatty ester comprises 40 ethylene oxide units.

4. Nanoemulsion according to any one of the preceding claims, **characterised in that** the ethoxylated fatty ester is polyethylene glycol stearate 40 EO and the fatty acid ester of sorbitan is sorbitan tristearate.

5. Nanoemulsion according to any one of the preceding claims, **characterized in that** the ionic surfactant is chosen from sodium palmitoyl sarcosinate.

6. Nanoemulsion according to any one of the preceding claims, **characterized in that** the weight ratio of the ionic surfactant (b) to the mixture of nonionic surfactants (a) is such that:
0.02 ≤ b/a ≤ 75 and preferably 0.02 ≤ b/a ≤ 10.

7. Nanoemulsion according to any one of the preceding claims, **characterized in that** the weight ratio of the ethoxylated fatty ester to the fatty acid ester of sorbitan ranges from 0.02 to 100 and preferably from 0.04 to 80.

8. Nanoemulsion according to any one of the preceding claims, **characterized in that** the fatty acid ester of sorbitan represents 0.1% to 10% by weight and preferably 0.5% to 5% by weight relative to the total weight of the nanoemulsion.

9. Nanoemulsion according to any one of the preceding claims, **characterized in that** the ethoxylated fatty ester represents from 0.01% to 10% by weight and preferably 0.1% to 5% by weight relative to the total weight of the nanoemulsion.

10. Nanoemulsion according to any one of the preceding claims, **characterized in that** the content of ionic surfactant represents from 0,05% to 10% and preferably 0.2% to 5% by weight relative to the total weight of the nanoemulsion.

11. Nanoemulsion according to any one of the preceding claims, **characterized in that** the only phase represents from 0.5% to 40% by weight and preferably from 5% to 30% by weight relative to the total weight of the nanoemulsion.

12. Nanoemulsion according to any one of the preceding claims, **characterised in that** the oily phase comprises an oil chosen from oils of animal or plant origin, mineral oil, synthetic oils, silicone oils and aliphatic hydrocarbons, and mixtures thereof.

13. Nanoemulsion according to any one of the preceding claims, **characterised in that** the oil is a hydrogenated polyisobutene, in particular parleam oil.

14. Nanoemulsion according to Claim 13, **characterized in that** it also comprises at least one other fatty substance chosen from fatty alcohols, fatty acids, waxes and gums, and mixtures thereof.

15. Nanoemulsion according to Claim 14, **characterized in that** the other fatty substance represents from 0% to 10% by weight and preferably 2% to 5% by weight relative to the total weight of the nanoemulsion.

16. Nanoemulsion according to Claim 13 or 14, **characterized in that** the fatty alcohols are chosen from stearyl alcohol, cetyl alcohol and behenyl alcohol, and the fatty acids are chosen from stearic acid, palmitic acid and behenic acid.

17. Nanoemulsion according to Claim 16, **characterized in that** the fatty alcohol is cetyl alcohol.

18. Nanoemulsion according to Claim 14 or 15, **characterized in that** the other fatty substance is glycerol mono-, di- or tripalmitostearate.

19. Nanoemulsion according to any one of the preceding claims, **characterized in that** it contains a cosmetic, dermatological or ophthalmological active agent.

20. Composition for topical use, **characterized in that** it contains a nanoemulsion according to any one of Claims 1 to 19.

21. Cosmetic use of the nanoemulsion according to any one of Claims 1 to 18, to care for, treat and/or make up the skin, the face and/or the scalp.

22. Cosmetic use of the nanoemulsion according to any one of Claims 1 to 18, to care for and/or treat the hair.

23. Use of the nanoemulsion according to any one of Claims 1 to 18, for the manufacture of a dermatological or ophthalmological composition,

24. Cosmetic process for caring for and/or moisturizing the skin, the mucous membranes and/or the scalp, **characterized in that** a nanoemulsion according to any one of Claims 1 to 13 is applied to the skin, the mucous membranes and/or the scalp.

25. Process for preparing a nanoemulsion according to any one of Claims 1 to 19, which consists in mixing together the aqueous phase and the oily phase, with vigorous stirring, at a temperature ranging from 60 to 95°C, and then in homogenizing at a pressure ranging from 6 × 10⁷ Pa to 18 x 10⁷ Pa.

26. Process according to Claim 25 **characterised in that** the shear ranges from 2 × 10⁶ s⁻¹ to 5 × 10⁵ s⁻¹.

## Patentansprüche

1. Nanoemulsion, die eine Ölphase enthält, die in einer wässerigen Phase dispergiert ist, die Ölkügelchen aufweist, wobei das Zahlenmittel der Größe der Ölkügelchen unter 100 nm liegt, **dadurch gekennzeichnet, dass** sie ein ternäres System von grenzflächenaktiven Stoffen enthalt:
(a) ein Gemisch aus mindestens zwei nichtionischen grenzflächenaktiven Stoffen, das zumindest einen ethoxylierten Fettsäureester mit 8 bis 100 Ethylenoxideinheiten und mindestens einen Sorbitanfettsäureester aufweist; und
(b) mindestens einen ionischen grenzflächenaktiven Stoff, der unter den Alkalisalzen von Palmitylsarcosinat ausgewählt ist.

2. Nanoemulsion nach Anspruch 1, **dadurch gekennzeichnet, dass** der ethoxylierte Fettsäureester 10 bis 80 Ethylenoxideinheiten ausweist.

3. Nanoemulsion nach Anspruch 1, **dadurch gekennzeichnet dass** der ethoxylierte Fettsäureester 40 Ethylenoxideinheiten aufweiset.

4. Nanoemulsion nach einem der vorhergehenden Ansprüche, **dadurch** dass es sich bei dem ethoxylierten Fettsäureester um das Polyethylenglycolstearat 40 EO und bei dem Sorbitanfettsäureester um das Sorbitantristearat handelt.

5. Nanoemulsion nach einem der vorhergehenden Ansprüche, **dadurch gekennzeichnet, dass** der ionische grenzflächenaktive Stoff das Natriumpalmitoylsarcosinat ist.

6. Nanoemulsion nach einem der vorhergehenden Ansprüche, **dadurch gekennzeichnet, dass** das Gewichtsverhältnis des ionischen grenzflächenaktiven Stoffes (b) und des Gemisches von nichtiomschen grenzflächenaktiven Stoffen (a) so ist, dass;
0,02 ≤ b/a ≤ 75 und vorzugsweise 0,02 ≤ b/a ≤ 10.

7. Nanoemulsion nach einem der vorhergehenden Ansprüche, **dadurch gekennzeichnet, dass** das Gewichtsverhältnis des ethoxylierten Fettsäureesters und des Sorbitanfettsäureesters im Bereich von 0,02 bis 100 und vorzugsweise 0,04 bis 80 liegt.

8. Nanoemulsion nach einem der vorhergehenden Ansprüche, **dadurch gekennzeichnet, dass** der Sorbitanfettsäureester 0,1 bis 10 Gew.-% und vorzugsweise 0,5 bis 5 Gew.-% des Gesamtgewichts der Nanoemulsion ausmacht.

9. Nanoemulsion nach einem der vorhergehenden Ansprüche, **dadurch gekennzeichnet, dass** der ethoxylierte Fettsäureester 0,01 bis 10 Gew. -% und vorzugsweise 0,1 bis 5 Gew.-% des Gesamtgewichts der Nanoemulsion ausmacht.

10. Nanoemulsion nach einem der vorhergehenden Ansprüche, **dadurch gekennzeichnet, dass** der Gehalt an ionischein grenflächenaktiven Stoff 0,05 bis 10 Gew.-% und vorzugsweise 0,2 bis 5 Gew.-% des Gesamtgewichts der Nanoemulsion ausmacht.

11. Nanoemulsion nach einem der vorhergehenden Ansprüche, **dadurch gekennzeichnet, dass** die Ölphase 0,5 bis 40 Gew.-% und vorzugsweise 5 bis 30 Gew.-%, bezogen auf das Gesamtgewicht der Nanoemulsion, ausmacht.

12. Nanoemulsion nach einem der vorhergehenden Ansprüche, **dadurch gekennzeichnet, dass** die Ölphase ein Öl enthalt, das unter den Ölen tierischer oder pflanzlicher Herkunft, Mineralölen,synthetischen Ölen, Siliconölen, aliphatischen Kohlenwasserstoffen und deren Gemischen ausgewählt ist.

13. Nanoemulsion nach einem der vorhergehenden Ansprüche, **dadurch gekennzeichnet, dass** es sich bei dem Öl um ein hydriertes Polyisobuten und insbesondere Parleamöl handelt.

14. Nanoemulsion nach Anspruch 13, **dadurch gekennzeichnet, dass** sie ferner mindestens eine Fettsubstanz enthält, die unter den Fettalkoholen Fettsäuren, Wachsen, Gummis und deren Gemischen ausgewählt ist.

15. Nanoemulsion nach Anspruch 14, **dadurch gekennzeichnet, dass** die weitere Fettsubstanz 0 bis 10 Gew.-% und vorzugsweise 2 bis 5 Gew.-%% des Gesamtgewichts der Nanoemulsion ausmacht.

16. Nanoemulsion nach Anspruch 13 oder 14, **dadurch gekennzeichnet, dass** die Fettalkohole unter Stearylalkohol, Cetylalkohol, Behenylalkohol und die Fettsäuren unter Stearinsäure, Palmitinsäure und Behensäure ausgewählt sind.

17. Nanoemulsion nach Anspruch 16, **dadurch gekennzeichnet, dass** der Fettalkohol der Cetylakohol ist.

18. Nanoemulsion nach, Anspruch 14 oder 15, **dadurch gekennzeichnet, dass** es sich bei der weiteren Fettsubstanz um Glycerylmono-, -di-, -tripalmitostearat handelt.

19. Nanoemulsion nach einem der vorhergehenden Ansprüche, **dadurch gekennzeichnet**, das sie einen kosmetischen, dermatologischen oder ophthalmologischen Wirkstoff enthält.

20. Zusammensetzung für die topische Anwendung, **dadurch gekennzeichnet dass** sie eine Nanoemulsion nach einem der Ansprüche 1 bis 19 enthalt.

21. Kosmetische Verwendung der Nanoemulsion nach einem der Ansprüche 1 bis 18 für die Pflege, die Behandlung und/oder das Schminken der Haut, des Gesichts und/oder der Kopfhaut

22. Kosmetische Verwendung der Nanoemulsion nach einem der Ansprüche 1 bis 18 für die Pflege und/oder die Behandlung der Haare.

23. Verwendung der Nanoemulsion nach einem der Anspruche 1 bis 18. für die Herstellung einer dermatologischen oder ophthalmologischen Zusammensetzung.

24. Kosmetisches Verfahren für die Pflege und/oder Hydratisierung der Haut, der Schleimhäute und/oder der Kopfhaut, **dadurch gekennzeichnet, dass** auf die Haut, die Schleimhäute und/oder die Kopfhaut eine Nanoemulsion nach einem der Ansprüche 1 bis 18 aufgetragen wird.

25. Verfahren zu Herstellung einer Nanoemulsion nach einem der Anspruche 1 bis 19, das darin besteht, die wässrige Phase und die Ölphase unter kräftigem Rühren bei einer Temperatur im Bereich von 60 bis 95°C zu vermischen und dann eine Homogenisierung bei einem Druck von 6 · 10⁷ bis 18 · 10⁷ Pa durchzuführen.

26. Verfahren nach Anspruch 35, **dadurch gekennzeichnet, dass** die Scherbeanspruchung im Bereich von 2 · 10⁶ bis 5 · 10⁶ s⁻¹ liegt.
